# EUROPEAN PATENT SPECIFICATION

(11) **EP 3 061 815 B1**
(45) Date of publication and mention of the grant of the patent: **19.02.2020**
(21) Application number: 14856578.1
(22) Date of filing: 24.10.2014
(51) Int. Cl.: C12N 9/12, A61K 38/00

(54) **PEPTIDES DERIVED FROM GSE 24.2 FOR TREATING DISEASES CAUSED BY OXIDATIVE STRESS AND DAMAGE TO DNA**
PEPTIDE AUS GSE 24.2 ZUR BEHANDLUNG VON ERKRANKUNGEN INFOLGE VON OXIDATIVEM STRESS UND DNA-SCHADEN
PEPTIDES DÉRIVÉS DE GSE 24.2 POUR TRAITER DES MALADIES ENGENDRÉES PAR LE STRESS OXYDATIF ET LES DOMMAGES À L'ADN

(30) Priority: 25.10.2013 ES 201331573
(43) Date of publication of application: 31.08.2016
(73) Proprietor: Consejo Superior de Investigaciones Científicas (CSIC), 28006 Madrid (ES); Universidad Autónoma de Madrid, 28049 Cantoblanco Madrid (ES); Advanced Medical Projects, 28760 Tres Cantos (Madrid) (ES); Centro de Investigación Biomédica en Red (CIBER), 28029 Madrid (ES)
(72) Inventor: PERONA ABELLÓN, Rosario, E-28029 Madrid (ES); SASTRE GARZÓN, Leandro, E-28029 Madrid (ES); PINTADO BERNINCHES, Laura, E-28029 Madrid (ES); CARRILLO GARCÍA, Jaime, E-28029 Madrid (ES); MOLINA PACHÓN, Antonio, E-28760 Tres Cantos (Madrid) (ES); IRRADICCIO SILVA, Laura, E-28760 Tres Cantos (Madrid) (ES); MANGUAN GARCÍA, Cristina, E-46010 Valencia (ES)
(74) Representative: Pons Ariño, Angel
(86) International application number: PCT/ES2014/070803
(87) International publication number: WO 2015/059338

(56) References cited:
- EP-A1- 1 947 175
- EP-A1- 2 216 043
- WO-A1-95/18857
- US-A- 5 942 389
- Anonymous: "Job result", , 13 August 2009 (2009-08-13), XP055417899, Retrieved from the Internet: URL:http://ibis/exam/jobResult?id=434041 [retrieved on 2017-10-23]

## Description

### Field of the invention

Peptides derived from GSE24.2 have applications in the biotechnology and pharmaceutical sectors for treating diseases and/or manifestations caused by and/or related to oxidative stress and/or damage to DNA such as inflammation or cellular ageing.

### State of the art

GSE24.2 is a peptide corresponding to the protein dyskerin, which is part of the telomerase complex. GSE24.2 is able to reactivate telomerase in pathological and physiological situations; in diseases with telomerase defects GSE24.2 activity increases the viability of cells derived from patients.

International patent application: WO2007/090911 A1 "Sequence of nucleotides and peptides GSE24.2 of dyskerin, which can induce telomerase activity, Method for obtaining same, Therapeutic compositions and Applications thereof" reveals the use of GSE24.2 for reactivating telomerase activity either in a medicine or a pharmaceutical composition. This international patent application identifies a sequence of 55 amino acids as the original functional unit. This invention describes new peptides of smaller size derived from the original sequence as well as new uses and applications thereof such as protection against oxidative stress and damage to DNA, both processes intimately related to inflammatory and cellular ageing processes.

Furthermore, this disclosure provides variants of these peptides derived from GSE24.2 that include nuclear localisation sequences enabling higher and better access to the nucleus, which is where they must exercise their biological activity, increasing their efficacy and reducing the need for encapsulation of the original sequence of 55 amino acids.

### Description of the invention

### Brief description of the invention

This disclosure provides a compound that reduces or decreases production of free radicals and/or damage to the structure of cellular DNA, where this compound is based on a fragment of the nucleotide or amino acid sequence of peptide GSE24.2 of dyskerin, capable of restoring normal cellular redox balance and/or correcting damage to cellular DNA. Thus, the invention addresses the problem of providing new therapeutic tools for the treatment of diseases involving an increase in cellular oxidative stress and/or damage to the structure of DNA such as arteriosclerosis, Parkinson's disease, myalgic encephalopathy, Alzheimer's disease, ataxia telangiectasia, congenital dyskeratosis and ageing.

This disclosure describes internal sequences of peptide GSE24.2 (SEQ ID No. 1) that have higher biological activity than the complete sequence and its already known fragments; furthermore, to improve their activity, nuclear transport sequences have been added that additionally improve the activity of the peptides by improving their internalisation by cells and increasing their presence in the cell nucleus.

Peptide GSE24.2 (with sequence SEQ ID No. 1) and derived peptides have biological activities not previously described such as the reduction of the free radical content by cells and therefore have activities of reducing oxidative stress and reducing structural damage to DNA caused by mutations in its recognition machinery; all this increases viability of cells exposed to various agents causing the effects previously mentioned. This activity has been tested in some pathologies such as ataxia telangiectasia and congenital dyskeratosis; but this also enables its application in neurodegenerative diseases, muscular degeneration (Duchenne dystrophy), progeroid syndromes, hypersensitivity to light, genetic instability caused by mutations or external agents; all applications not previously described for this peptide.

### Detailed description of the invention

The basis of this disclosure is that the inventors have tested the ability of peptides derived from GSE24.2 for reducing the level of free radicals and the damage to DNA present in various human diseases or derived from exposure to agents such as radiation or genotoxic agents. Therefore, peptides derived from GSE24.2 of this disclosure as well as GSE24.2 itself can be applied to all these diseases caused or involving an accumulation of damage to DNA or an increase in free radicals such as: diseases of accelerated ageing such as Werner syndrome or Hutchison-Gilford syndrome; diseases where there is damage to DNA caused by mutations in the recognition and processing machinery such as ataxia telangiectasia, xeroderma pigmentosum, Rothmund-Thomson syndrome, Nijmegen breakage syndrome; neurodegenerative diseases such as for example, Huntington's, Alzheimer's, Parkinson's diseases; diseases involving muscular degeneration such as Duchenne muscular dystrophy; cardiovascular or lung diseases such as pulmonary fibrosis or arteriosclerosis; also diseases involving a major inflammatory component in their pathology such as rheumatoid arthritis. Applications of these peptides also include those related to tissue engineering and cell culture and other biotechnological applications. These peptides may also have applications in the cosmetics industry such as, for example, in the treatment of damage caused by ageing in the skin.

The inventors have produced small sized peptides derived from GSE24.2 (SEQ ID No. 1) with the ability to enter the cell and that have higher biological activity than the original peptide, identifying them in bacterial lysates of bacteria that express the full sequence of GSE24.2. These lysates were analysed by HPLC prior to the identification of their sequences. Five (5) peptides in addition to GSE24.2 were identified in lysates and their sequences corresponded to SEQ ID No. 2, SEQ ID No. 3, SEQ ID No. 4, SEQ ID No. 5, SEQ ID No. 6.

Table 1 shows a listing of the sequences and correspondences with the peptides described in this disclosure.

**Table 1. Correspondence between the description of the peptides and the sequences used in the description.**

| **Name** | **Sequence** | **No. amino acids** | **SEQ ID No.** |
|---|---|---|---|
| GSE24.2 | | 55 | 1 |
| GSE4 | GFINLDKPSNP | 11 | 2 |
| GSE1 | GFINLDKPSNPSSHEVV | 17 | 3 |
| GSE2 | GFINLDKPSNPSSHEVVA | 18 | 4 |
| GSE3 | GFINLDKPSNPSSHEVVAW | 19 | 5 |
| GSE5 | | 25 | 6 |
| GSE4NLS1-3 | GFINLDKPSNPKRKR | 15 | 7 |
| GSE24.2NLS1-3 | | 59 | 8 |
| GSE24.2NLS2-3 | | 63 | 9 |
| GSE24.2NLS1-5 | | 59 | 10 |
| GSE24.2NLS2-5 | | 63 | 11 |
| GSE4NLS1-5 | KRKRGFINLDKPSNP | 15 | 12 |
| GSE4NLS2-3 | GFINLDKPSNPKKEKKKSK | 19 | 13 |
| GSE4NLS2-5 | KKEKKKSKGFINLDKPSNP | 19 | 14 |
| SGSE4 | HSGTLDPKVTG | 11 | 15 |

The biological activity of the peptides identified in the bacterial lysates was studied in a DNA damage repair test. Of these, peptide GSE4 (SEQ ID No. 2), which is included in the TRUBL domain, substantially improved the activity of the full domain (SEQ ID No. 4, GSE2) and of GSE24.2 (SEQ ID No. 1); this activity was still further improved by the insertion of a sequence for nuclear localisation in the carboxyl terminal position (but not in the N-terminal position). Given the small size of the peptides identified in this disclosure, it is not necessary to use cell capture systems such as liposomes to introduce them into the cell; this is a substantial improvement compared to that used for the full peptide GSE24.2 (SEQ ID No. 1) or for the TRUBL domain (GSE2, SEQ ID No. 4) where liposomes are required. These new peptides, therefore, provide a more reproducible and stable system for therapeutic purposes than the current. Furthermore, these peptides can be obtained by chemical synthesis, simplifying the process of obtaining them. These peptides have functions analogous to those of GSE24.2 and therefore it is possible to use them in equivalent applications.

This disclosure identifies the minimum unit or minimum polypeptide with activity that is included in the GSE24.2 peptide of sequence SEQ ID No. 1 and that consists of a fragment of 11 amino acids of SEQ ID No. 1 and that comprises at least SEQ ID No. 2. This peptide constitutes the minimum unit responsible for GSE24.2 activity of reducing free radical production and/or damage to the structure of DNA in a cell.

Thus, the present disclosure refers to a polypeptide comprising at least a fragment of the sequence of the peptide GSE24.2 with SEQ ID No. 1 able to reduce the production of free radicals and/or the damage to cellular DNA structure and where this fragment comprises, at least, SEQ ID No. 2, except when this polypeptide consists of the peptide GSE24.2 (SEQ ID No. 1) or a fragment of it with sequence SEQ ID No. 4 (GSE2). Prefrably, the polypeptide has an amino acid sequence that comprises a fragment of the peptide GSE24.2 selected from at least one of the group formed by: SEQ ID No. 2, SEQ ID No. 3, SEQ ID No. 5 and SEQ ID No. 6.

Accordingly, a first aspect of the present invention relates to a polypeptide comprising at least a fragment of the sequence of the peptide GSE24.2 (SEQ ID No. 1) with SEQ ID No. 2 able to reduce production of free radicals and/or damage to the structure of cellular DNA and said polypeptide that is selected from the list consisting of: SEQ ID No. 2, SEQ ID No. 5 and SEQ ID No. 6.

When at least one of the polypeptides previously defined are introduced or its expression is induced into the interior of the cell, preferably into the interior of a eukaryotic cell such as a human cell, it is able to cause a reduction in the production of free radicals and/or damage to the DNA of this cell. In this way, on quantifying the levels of free radicals in a live cell by any of the known methods such as for example by the compound dihydroethidium (DHE, Hydroethidine), the production of free radicals in the cell can fall depending on the sequence of this polypeptide until reaching physiological levels with respect to a cell of the same type to which it has not been introduced or in which its expression has not been induced. Similarly, when the damage to cellular DNA is quantified by any of the existing methods such as, for example, the study of the hybridisation signal of cells with an antibody against phosphorylated histone H2AX in position Ser139 (γ-H2AX), this damage to DNA can be observed to reduce by 50% depending on the sequence of the polypeptide with respect to a cell of the same type to which it has not been introduced or in which its expression has not been induced.

Taking into account that the action of the polypeptides defined in this aspect of the invention can take place in the cell nucleus, in order to facilitate their expression or localisation in the nucleus and/or improve the activity of the polypeptides, this polypeptide, apart from the fragment of the GSE24.2 peptide with activity, can also comprise at least a nuclear localisation sequence (NLS) linked to at least one of the carboxyl and amino terminal ends of this fragment, and more preferably to the carboxyl terminal end.

In the scope of this first aspect, the nuclear localisation sequence is considered to be a group of amino acids capable of directing the localisation of the peptide to be bound to the cell nucleus; this sequence can be either a sequence for transport to the nucleus or a sequence for transport to the nucleolus. Preferably, the nuclear localisation sequence is a nuclear localisation sequence that is found in gene DKC1 from which the sequence of GSE24.2 is derived such as, for example, the nuclear transport sequence KRKR (NLS1) (SEQ ID No: 16) or the nucleolar transport sequence KKEKKKSK (NLS2) (SEQ ID No: 17), and more preferably it is the nuclear transport sequence KRKR (NLS1) (SEQ ID No: 16).

Thus, in a preferred embodiment of the polypeptide of this aspect of the invention, when it comprises a nuclear localisation sequence linked to the active fragment of GSE24.2, this polypeptide consists of an amino acid sequence selected from the group composed of: SEQ ID No. 7, SEQ ID No. 12, SEQ ID No. 13 and SEQ ID No. 14.

Throughout this document, any of the polypeptides defined in this first aspect of the invention can be equally referred to as "polypeptide of the invention" or "peptide of the invention".

The polypeptides of the invention can be obtained by the normal procedures known in the art for peptide synthesis: by insertion into expression vectors or by chemical synthesis. Chemical synthesis procedures of peptides comprise both synthesis in solution or liquid phase and solid phase peptide synthesis (SPPS). Preferably, solid phase peptide synthesis would be used, using any of the methods known in the state of the art such as Fmoc and t-Boc.

Another aspect of the invention refers to a polynucleotide that comprises a sequence that codes for at least one of the polypeptides of the invention such as any of those previously defined. This polynucleotide, also referred to in this document as polynucleotide of the invention, enables the expression of a polypeptide that is able to reduce production of free radicals and/or damage to the structure of DNA in a eukaryotic cell, preferably a human cell, and can refer to a polynucleotide of DNA, cDNA or RNA.

In a preferred embodiment, the polynucleotide of the invention comprises a sequence that codes for the polypeptide of the invention selected from at least one of the group formed by: SEQ ID No. 2, SEQ ID No. 5 and SEQ ID No. 6.

Taking into account that the action of the polypeptide that is coded by the polynucleotide of the invention can take place in the cell nucleus, similarly as indicated for the polypeptide of the invention, the polynucleotide sequence of the invention can also comprises at least one nuclear localisation sequence (NLS) bound to at least one of the 3' or 5' ends of the sequence of the fragment of peptide GSE24.2, and more preferably bound to the 3' end.

Preferably, the nuclear localisation sequence chosen for the polypeptide of the invention is a nuclear localisation sequence directed at the nucleus or at the nucleolus, and more preferably, it is in the gene DKC1 from which the sequence of GSE24.2 is derived. Preferred examples of nuclear transport sequences of gene DKC1 are the nuclear transport sequences that code for the peptide sequence KRKR (NLS1) (SEQ ID No: 16) or the nucleolar transport sequences that code for the peptide sequence KKEKKKSK (NLS2) (SEQ ID No: 17), and preferably it is a nuclear transport sequence that codes for the peptide sequence KRKR (NLS1) (SEQ ID No: 16).

In a preferred embodiment, when the polynucleotide of the invention comprises a nuclear localisation sequence linked to the sequence coding for the active fragment of GSE24.2, this polynucleotide consists of a sequence that codes for a polypeptide selected from the group composed of SEQ ID No. 7, SEQ ID No. 12, SEQ ID No. 13 and SEQ ID No. 14.

The polynucleotide of the invention as any of those described above can be obtained by an expert through the use of techniques widely known in the state of the art (Sambrook et al. "Molecular cloning, a Laboratory Manual" 2nd ed., Cold Spring Harbor Laboratory Press, N.Y., 1989 vol 1-3). The sequences of these polynucleotides can be integrated in a gene expression vector that enables regulation of expression in suitable conditions inside the cells. Therefore, another aspect of the invention refers to an expression vector that comprises a polynucleotide of the invention or a polynucleotide sequence that codes for a polypeptide of the invention and that enables the expression of a polypeptide that is able to reduce the production of free radicals and/or damage to the structure of DNA in a eukaryotic cell, preferably a human cell, hereafter the vector of the invention.

In general, according to this description, the expression vector further comprises, in addition to the polynucleotide described in the invention, a promoter that directs its transcription (for example, pT7, plac, ptrc, ptac, pBAD, ret, etc.), to which it is operationally linked, and other necessary or appropriate sequences that control and regulate this transcription and, if applicable, the translation of the product of interest, for example, transcription initiation and termination signals (tlt2, etc.), polyadenylation signal, replication origin, ribosome binding sequences (RBS), sequences encoding transcription regulators (enhancers), transcription silencers (silencers), repressors, etc. Examples of suitable expression vectors can be selected in accordance with the conditions and needs of each specific case from expression plasmids, viral vectors (DNA or RNA), cosmids, artificial chromosomes, etc. that may additionally contain labels used for selecting cells transfected or transformed with the gene or genes of interest. The choice of vector will depend on the host cell and the type of use to be made of it. Therefore, according to a particular embodiment of this description, this vector may be a plasmid or viral vector. This vector may be obtained by conventional methods known to those skilled in the subject in the same way that for the transformation of micro-organisms and eukaryotic cells various widely known methods may be used: chemical transformation, electroporation, microinjection, etc., described in various manuals (Sambrook et al. "Molecular cloning, a Laboratory Manual" 2nd ed., Cold Spring Harbor Laboratory Press, N.Y., 1989 vol 1-3).

The polypeptide of the invention, the polynucleotide of the invention and/or the gene expression vector defined in this invention can be used as a medicine in a treatment and/or prevention procedure for a human being affected by a disease caused by an increase in oxidative stress and/or damage to cellular DNA, preferably through its biological action in cells affected by an increase in free radical levels and/or damage to the structure of their DNA, preferably human cells; it may also be used as a biotechnological tool in cell culture systems or *in vitro* tissue engineering to improve cell viability. However, this disclosure also considers that the complete sequence of peptide GSE24.2 (SEQ ID No. 1) and/or its 18-amino acid fragment SEQ ID No. 4 (GSE2) can be used for this purpose. It is considered that biotechnological and pharmaceutical tools as well as therapy procedures described in this description are sufficiently known by an expert in the art so that such tools can be developed without excessive effort.

Thus another aspect of the invention refers to the use of a polypeptide, or of a polynucleotide or vector that codes for this polypeptide, and is able to reduce the production of free radicals and/or damage to the structure of cellular DNA, in a medicine preparation or pharmaceutical composition, for the treatment of, at least, one disease caused by an alteration, preferably an increase, of oxidative stress and/or damage to cellular DNA. By way of an illustration and without limiting the scope of the invention, this disease may belong to the following group: congenital dyskeratosis, a neurodegenerative disease (such as Alzheimer's, Parkinson's, Huntington's, cerebellar ataxia or spinal medulla degeneration), Cri du Chat syndrome, ataxia telangiectasia, Nijmegen breakage syndrome, Bloom syndrome, Werner syndrome, Fanconi anaemia, ulcerative colitis, vascular ageing, arteriosclerosis, preferably atherosclerosis, cancer, Duchenne muscular dystrophy, progeroid syndromes, light hypersensitivity, genetic instability caused by mutation or external agent, Hutchison-Gilford syndrome, xeroderma pigmentosum, Rothmund-Thomson syndrome, pulmonary fibrosis and diseases with chronic inflammation such as, for example, rheumatoid arthritis.

According to an aspect of the invention, the medicine or pharmaceutical composition comprises a polypeptide and/or polynucleotide or expression vector that codes for it, where this polypeptide comprises a sequence selected from, at least, one of the group composed of SEQ ID No. 2, SEQ ID No. 5 and/or SEQ ID No. 6.

In another aspect of the invention, the medicine or pharmaceutical composition comprises a polypeptide and/or a polynucleotide or expression vector that codes for it, where the polypeptide additionally comprises at least one nuclear localisation sequence, preferably bound at the carboxyl terminal end, preferably selected from at least one of the group composed of SEQ ID No. 7, SEQ ID No. 12, SEQ ID No. 13, and SEQ ID No. 14.

Another object of the invention is a pharmaceutical composition or medicine, preferably for the treatment of diseases, disorders or pathologies caused by a change in the content of free radicals inside the cell, causing oxidative stress associated with and/or caused by damage to the structure of cellular DNA, hereafter the pharmaceutical composition of the invention, that comprises at least one polypeptide, polynucleotide and/or expression vector able to reduce the production of free radicals and/or damage to the structure of cellular DNA, in a therapeutically effective amount together with, optionally, one or more adjuvants and/or pharmaceutically acceptable vehicles, and that is able to regulate and maintain the redox balance and correct damage to DNA. In the scope of this invention, this pharmaceutical composition or medicine can equally be referred to as the "pharmaceutical composition or medicine of the invention".

The pharmaceutically acceptable adjuvants and vehicles that can be used in these compositions are adjuvants and vehicles known by experts in the field and commonly used in the preparation of therapeutic compositions. By way of example, these may be nanoparticles or liposomes, which help absorption and internalisation of molecules.

In the meaning used in this description, the expression therapeutically effective amount refers to the amount of polypeptide of the invention, or of a molecule or compound that enables the expression of a nucleotide sequences that codes for this polypeptide, able to reduce production of free radicals and/or damage to the structure of cellular DNA, calculated to cause the desired effect and that, in general, would be determined, among other factors, by the properties of the compounds, including age, patient status, severity of the alteration or disorder, and the route and frequency of administration.

In a particular embodiment, this therapeutic composition is prepared in the form of a solid or aqueous suspension in a pharmaceutically acceptable diluent. The therapeutic composition provided by this invention can be administered by any appropriate administrative route, so the composition would be formulated in a suitable pharmaceutically acceptable form for the chosen administration route. In a particular embodiment, administration of the therapeutic composition provided by this invention is by the parenteral route, oral route, intraperitoneal route or subcutaneous route. A review of various pharmaceutical forms of administration of medicines and of the excipients necessary for obtaining them can be found, for example, in Tratado de Farmacia Galénica, C. Fauli i Trillo, 1993, Luzán 5, S.A. Ediciones, Madrid.

In a preferred embodiment, the pharmaceutical composition or medicine comprises a polypeptide of the invention that is selected from at least one of the group composed of SEQ ID No. 2, SEQ ID No. 5, SEQ ID No. 6, SEQ ID No. 7, SEQ ID No. 12, SEQ ID No. 13 and SEQ ID No. 14; or a polynucleotide or vector that codes for this polypeptide.

The pharmaceutical composition of this invention can be used in a treatment method either alone or together with other pharmaceutical compounds.

Another aspect of the description is therefore the use of the pharmaceutical composition of the invention, hereafter the use of the pharmaceutical composition of the invention, in a method of treatment or prevention in an individual, preferably a human being, affected by a disease, disorder or pathology caused by an alteration in the content of free radicals inside the cell, causing oxidative stress and/or caused by damage to the structure of DNA. Preferably, the disease or pathology to be treated is one of those defined in the uses of the composition mentioned above.

Another aspect of the invention refers to the use of a polypeptide, or of the polynucleotide or vector that codes for it, in tissue engineering and cell culture to improve cell viability. Preferably, this polypeptide consists of at least one selected from the group composed of: SEQ ID No. 2, SEQ ID No. 5, SEQ ID No. 6, SEQ ID No. 7, SEQ ID No. 12, SEQ ID No. 13 and SEQ ID No. 14.

Throughout the description and the claims, the word "comprises" and its variants does not exclude other technical characteristics, additives, components or steps. For experts in the field, other objects, advantages and characteristics of the invention will emerge, partly from the description and partly from the practice of the invention. The following examples and figures are provided for illustration purposes only and are not to be considered as limiting the present invention.

### Brief description of the figures

**FIG. 1****.** Determination of damage to DNA in AT cells: AT3784 AT cells were infected with a lentiviral vector expressing GSE24.2 or a GFP empty vector and were fixed 24 hours later to be processed and hybridised with an antibody against phosphorylated histone H2AX (Ser139). Control C-1787 cells were used as the negative control. Next they were hybridised with a fluorescein-labelled antibody and were stained with DAPI to locate the nuclei. The foci were evaluated with a stain for phosphorylated histone H2AX in 200 cells per type and the results were grouped into cells that did not show foci, foci numbers between 5 and 20 and foci numbers greater than 20.
**FIG. 2****.** Determination of damage to DNA in AT cells: AT3784 AT cells were infected with a lentiviral vector expressing GSE24.2 or a GFP empty vector and were fixed 24 hours later to be processed and hybridised with an antibody against the kinase pCHK2thr68. Control C-1787 cells were used as the negative control. Next they were hybridised with a fluorescein-labelled antibody and were stained with DAPI to locate the nuclei. The foci were evaluated with stain for pCHK2thr68 in 200 cells per type and the results were grouped into cells that did not show foci, foci numbers between 5 and 20 and foci numbers greater than 20.
**FIG. 3****.** Determination of ROS levels in AT cells: AT3189-P AT cells were infected with a lentiviral vector expressing GSE24.2 or a GFP empty vector and three weeks later were loaded with the DHE reagent that detects Reactive Oxygen Species (ROS) levels. AT736C cells were used as a control. Relative fluorescence of the three cell lines and the proportion of cells with maximum intensity were determined through flow cytometry using wavelengths of 518 nm absorption and 605 nm emission. The data are shown as relative percentage of cells that emit at this wavelength.

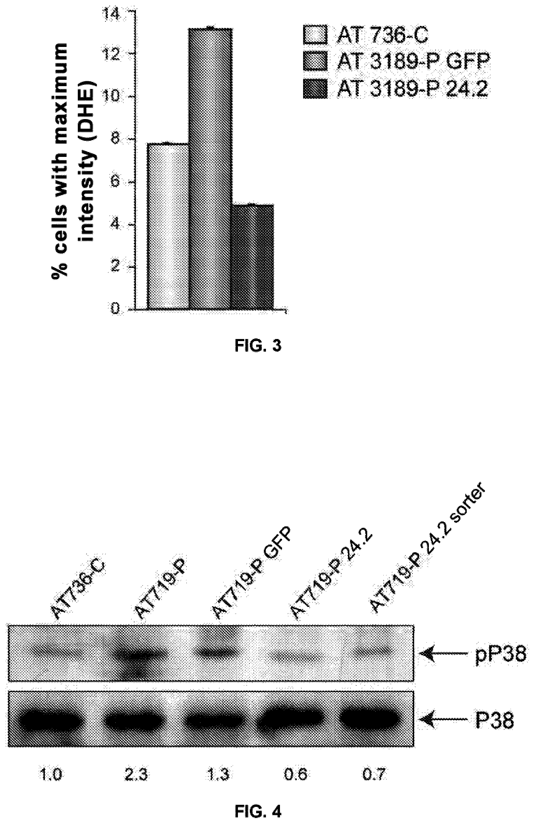

**FIG. 4****.** Determination of the levels of phosphorylation of p38 in AT cells: AT719-P AT cells were infected with a lentiviral vector expressing GSE24.2 or a GFP empty vector and three weeks later protein extracts were obtained. AT719-P AT cells infected with GSE24.2 were also used and the population with the maximum fluorescence was selected using a sorter (AT AT719-P GSE24.2 sorter). AT736C cells were used as a control. The proteins were resolved on polyacrylamide gels and transferred to nitrocellulose membranes. The membrane was hybridised with a polyclonal antibody against the protein p38 phosphorylated on Thr180/Try182 and non-phosphoryated protein as a loading control. The relative values between the phosphorylated and non-phosphorylated proteins obtained (using the NIH-Image program) for the control cell line were considered as 1 and the others referred to it.

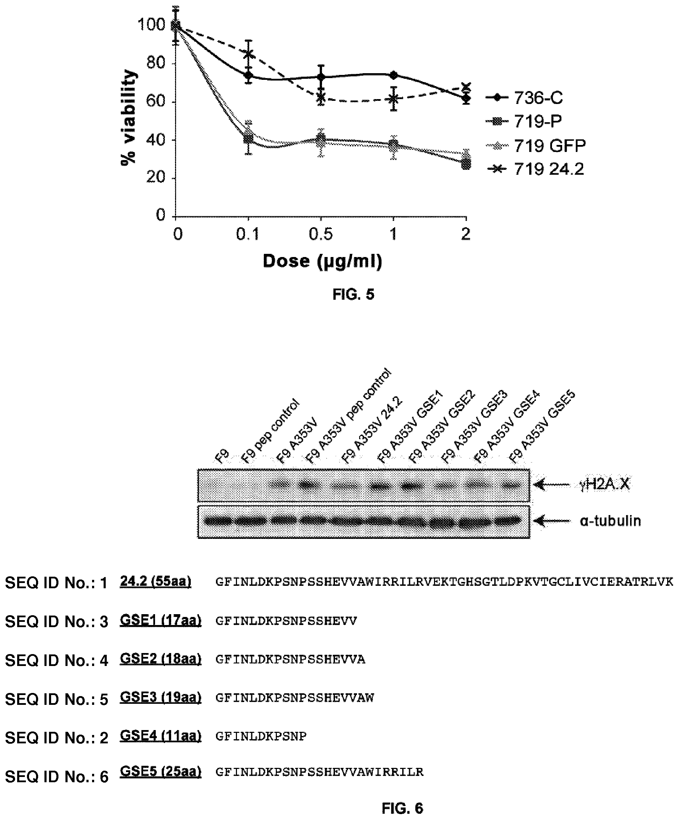

**FIG. 5****.** Determination of the viability in response to bleomycin in AT cells: AT719-P AT cells were infected with a lentiviral vector expressing GSE24.2 or a GFP empty vector and three weeks later they were treated with different doses of bleomycin. AT736C cells were used as a control. The cells were fixed after 72 hours of treatment and viability determined with the MTS reagent. The values represent the result of the test performed in triplicate and express the viability with respect to untreated cells.
**FIG. 6****.** Western blot of F9 and F9A353V cells after 24 hours of transfection with chemically synthesized peptides derived from GSE24.2. The peptides indicated on each line of gel were transfected (15 µg of each peptide) on a 30 mm plate. After separating the protein extracts on polyacrylamide gels and transferring them to a nitrocellulose membrane, the membrane was hybridised with an antibody against histone H2AX (Ser139) and then with an antibody against α-tubulin as a loading control.

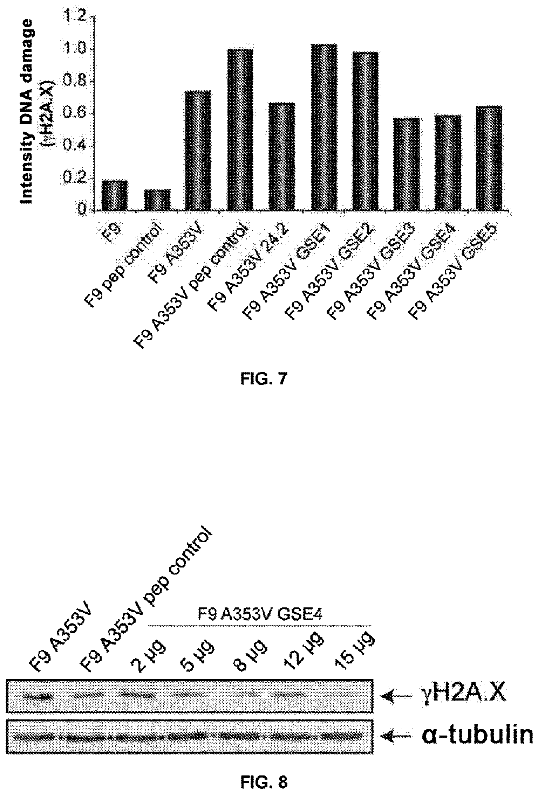

**FIG. 7****.** Quantification of Western blot of Figure 6. The signal obtained with histone H2AX (Ser139) was corrected with the signal of α-tubulin considering the signal obtained with the F9 cells transfected with beta-galactosidase (as control) as 1, using the NIH-Image program.
**FIG. 8****.** Western blot of F9 and F9A353V cells after 24 of transfection with the GSE4 peptide at increasing concentrations. The GSE4 synthetic peptide was transfected at the concentrations indicated on each line of the gel. The membrane was hybridised with an antibody against histone H2AX (Ser139) and α -tubulin as loading control.

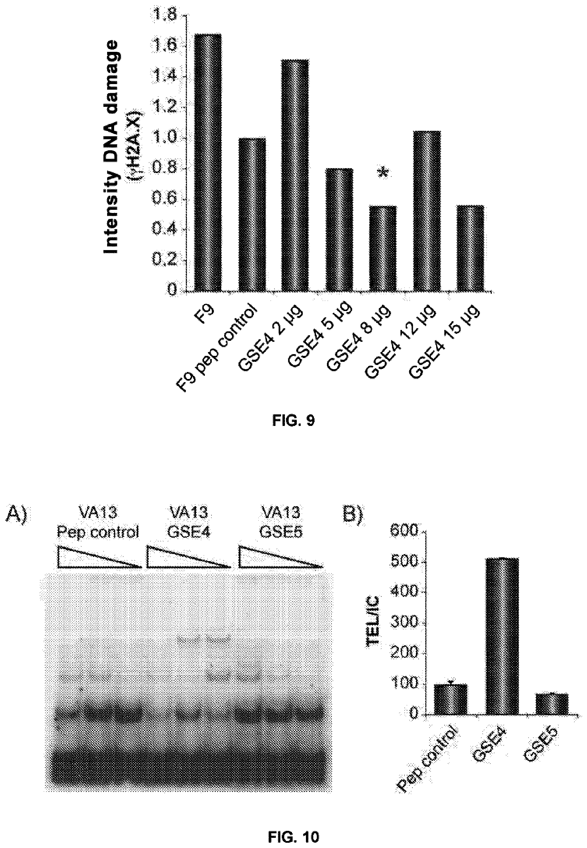

**FIG. 9****.** Quantification of the Western blot of Figure 8. The signal obtained with histone H2AX (Ser139) was corrected with the α-tubulin signal, considering the signal obtained with F9 cells transfected with beta-galactosidase as 1.
**FIG. 10****.** Determination of telomerase activity in VA13 cells after transfection of the peptides GSE4 and GSE5. In order to determine the effect of expression of peptides GSE4 and GSE5, VA13 cells were transfected through lipofection and 24 hours later a TRAP assay was performed for telomerase activity.

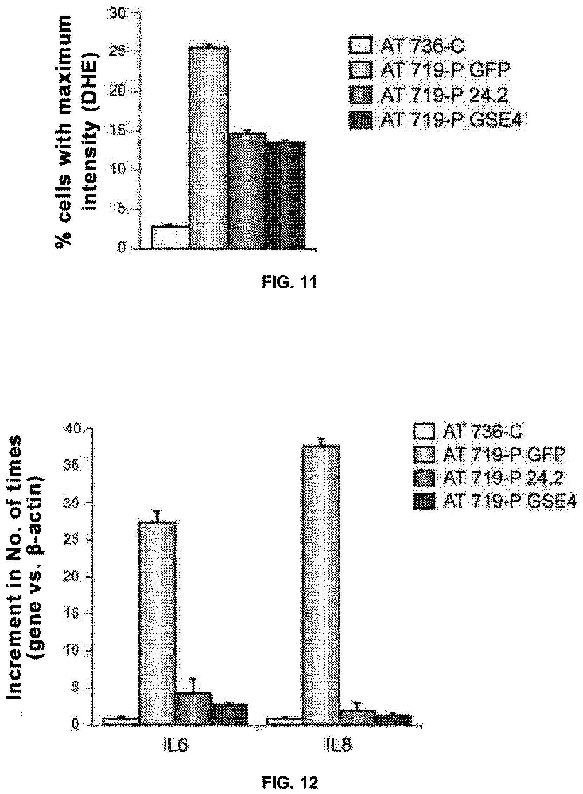

**FIG. 11****.** Determination of oxidative stress levels in ataxia telangiectasia cells after expressing the peptides GSE24.2 and GSE4. AT719-P cells were infected with a lentiviral vector expressing GSE24.2, GSE4 or a GFP empty vector and three weeks later loaded with the DHE reagent that detects levels of reactive oxygen species (ROS). AT736C cells were used as a control. The relative fluorescence of the four cell lines and the proportion of cells with maximum intensity were determined by flow cytometry at wavelengths of 518 nm absorption and 605 nm emission. The data are shown as relative percentage of cells that emit at this wavelength.
**FIG. 12****.** Levels of expression of proinflammatory cytokines in ataxia telangiectasia cells after expressing peptides GSE24.2 and GSE4. AT719-P AT cells were infected with a lentiviral vector expressing GSE24.2, GSE4 or a GFP empty vector and three weeks later the RNA was extracted and after obtaining the corresponding cDNA, the levels of expression of the proinflammatory cytokines IL6 and IL8 were determined. AT736C cells were used as a control. The levels of expression of the gene β-actin were measured as control of expression. The results are expressed as the relative increase in the number of times of expression of each of the genes compared to β-actin, considering the levels of expression of AT736C cells as 1.
**FIG. 13****.** Determination of senescence of AT cells after expressing peptides GSE24.2 and GSE4: AT2078 AT cells were infected with a lentiviral vector expressing GSE24.2, GSE4 or a GFP empty vector and 3 days or eight days later they were fixed to determine the expression of the enzyme β-galactosidase acid associated with senescence (senescence-associated β-galactosidase). Control C-1787 cells were used as the negative control. The percentage of cells expressing β-galactosidase from the total cells per field was determined. 200 cells per cell type were evaluated and the results grouped into cells showing no activity (βgal-, in division) and senescent cells (β-gal+).

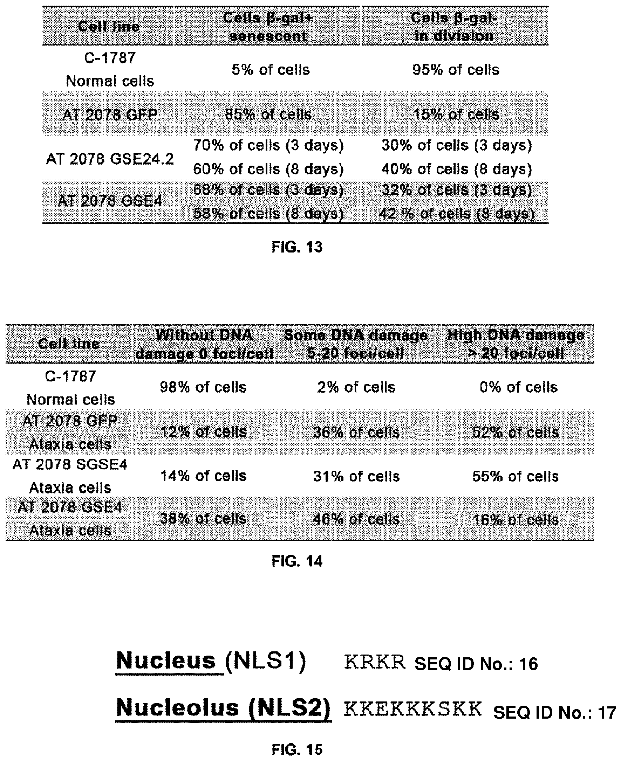

**FIG. 14****.** Determination of damage to DNA in AT cells after transfecting peptides GSE4 and SGSE4: AT2078 AT cells were infected with a lentiviral vector expressing GSE4, SGSE4 or a GFP empty vector and 24 hours later were fixed for processing and hybridised with an antibody against phosphorylated histone H2AX (Ser139). Control C-1787 cells were used as the negative control. Next they were hybridised with a fluorescein-labelled antibody and were stained with DAPI to locate the nuclei. The foci were evaluated with a stain for phosphorylated histone H2AX in 200 cells per type and the results were grouped into cells that did not show foci, foci numbers of between 5 and 20 and foci numbers greater than 20.
**FIG. 15****.** Sequences NLS1 (SEQ ID No: 16) and NLS2 (SEQ ID No: 17) of the sequence of dyskerin. Nuclear localisation sequence (NLS1) and nucleolar localisation sequence (NLS2) contained in the gene DKC1.

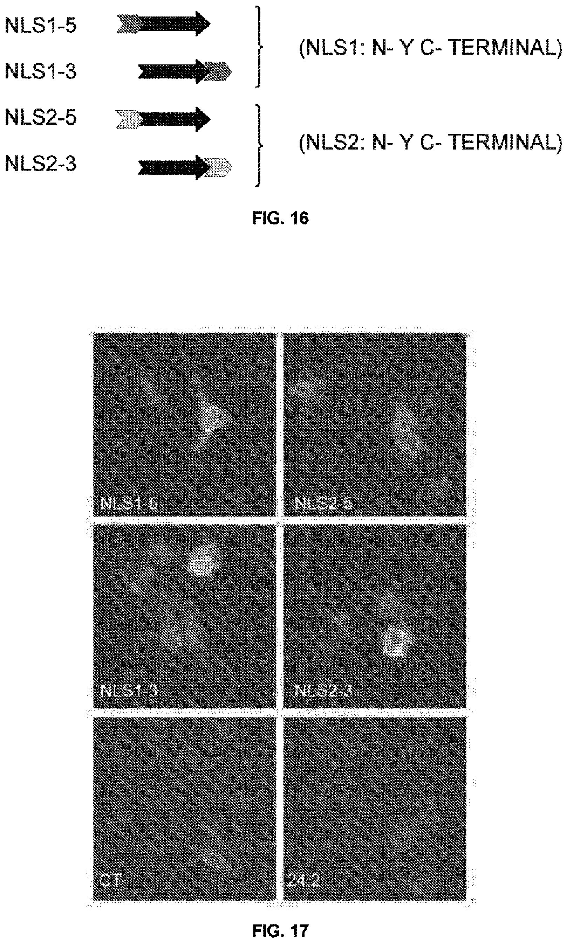

**FIG. 16****.** Schema of the constructions used for placing nuclear localisation signals NLS1 or NLS2 in positions 5' or 3' in the sequence of GSE24.2. Four (4) constructions were obtained, placing the NLS1 or NLS2 sequences in region 5' or 3' of GSE24.2 containing a tag for myc in the expression vector pCDNA3myc.
**FIG. 17****.** Expression of constructions NLS1-3, NLS1-5, NLS2-3 and NLS2-5 GSE24.2 and GSE24.2 in HeLa cells. Twenty-four (24) hours after tranfecting the plasmids (using lipofectamine) subcellular expression and localisation of the peptides was detected by immunofluorescence using an antibody against the c-myc tag present in the constructions (see Fig. 16).

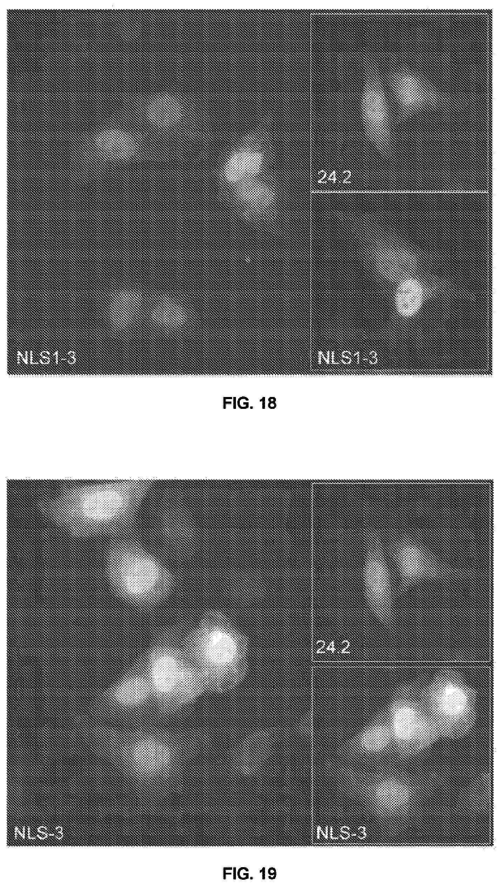

**FIG. 18****.** Expression of constructions NLS1-3 GSE24.2 and GSE24.2 in HeLa cells. 24 hours after transfecting the plasmids (using lipofectamine), the subcellular expression and localisation of the peptides were detected by immunofluorescence with an antibody against the c-myc tag that was present in the construction.
**FIG. 19****.** Expression of constructions NLS2-3 GSE24.2 and GSE24.2 in HeLa cells. Twenty-four (24) hours after transfecting the plasmids (using lipofectamine), the subcellular expression and localisation of the peptides were detected by immunofluorescence with an antibody against the c-myc tag that was present in the construction.

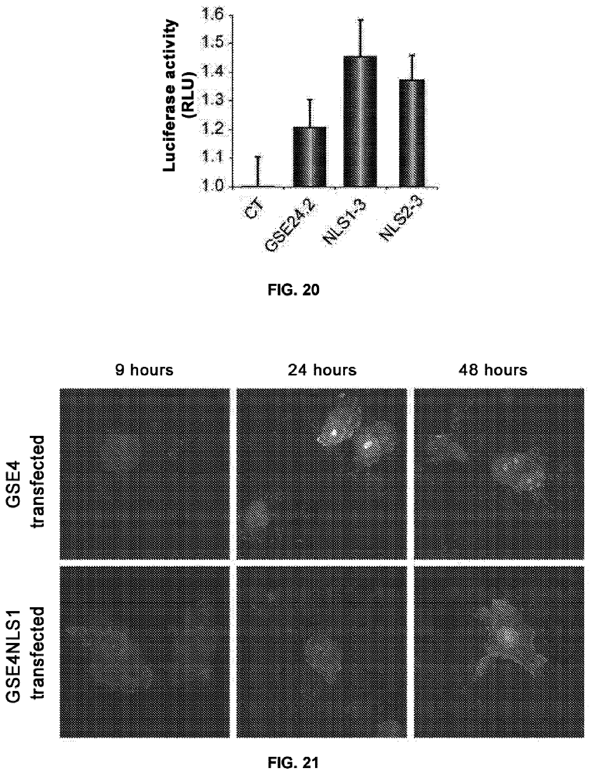

**FIG. 20****.** Activity of constructions GSE24.2NLS1-3 and GSE24.2NLS2-3 on the c-myc promoter. The expression vectors for the constructions GSE24.2, GSE24.2NLS1-3 and GSE24.2NLS2-3 were transfected (using lipofectamine) into 293T cells together with the reporter gene of the promoter of c-myc, pXP1, and 24 hours later the luciferase activity was measured using a commercial kit. The values are expressed as relative activity, considering 1 as the value of luciferase with the empty expression vector pCDNA3myc.
**FIG. 21****.** Transfection of peptides GSE4 and GSE4NLS1 in HeLa cells. Peptides GSE4 and GSE4NLS1 (SEQ ID No. 7) labelled with fluorescein and obtained by chemical synthesis were transfected into HeLa cells using the Proteojuice reagent and viewed in a confocal fluorescence microscope at 9, 24 and 48 hours after transfection.

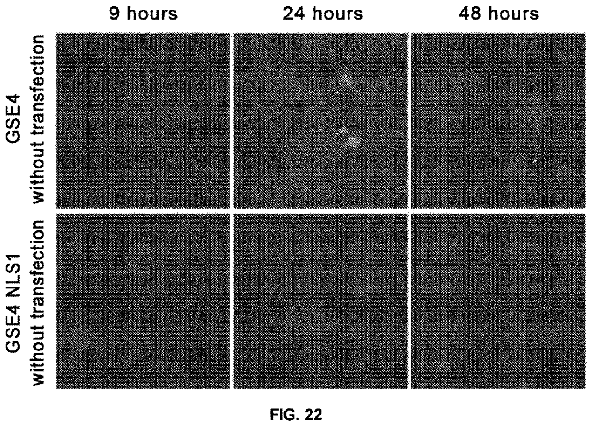

**FIG. 22****.** Internalisation of peptides GSE4 and GSE4NLS1 in HeLa cells. Peptides GSE4 and GSE4NLS1 (SEQ ID No. 7) labelled with fluorescein and obtained by chemical synthesis were added to the culture medium of HeLa cells and viewed in a confocal fluorescence microscope at 9, 24 and 48 hours after transfection.

### Examples

### EXAMPLE 1. Ability of GSE24.2 to reverse damage to DNA in cells from patients with ataxia telangiectasia.

To perform this test, cell lines from patients with ataxia telangiectasia (AT, from the Coriell repository) with a mutation in the gene ATM and denominated AT3487 were used. Cell line C-1787 was used as the control cell line. The cells were infected with the bicistronic lentiviral vector pCMV-GFP-GSE24.2. After 48 hours of infection the cells were fixed with formaldehyde and then permeabilized by treating them with Triton X-100. The damage to DNA was estimated by studying the signal after hybridisation of the cells with an antibody against histone H2AX Ser139 and subsequent hybridisation with an antibody bound to fluorescein. The number of foci with stain for histone H2AX/nucleus was determined by confocal microscopy in a total of 200 cells and the data represented as a percentage of cells with a specific number of foci.

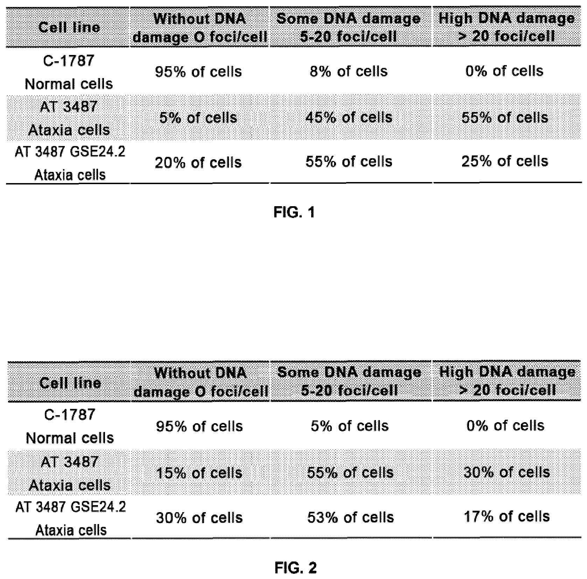

Figure 1 shows that the expression of GSE24.2 fully corrects the overall damage to DNA in 20% of the AT cells and reduces the levels of damage to DNA in 25% of AT cells. Therefore there was a correction of overall damage to DNA of 40%.

Next, a similar experiment was carried out but determining the damage to DNA by hybridisation with an antibody that recognises protein pCHK2 phosphorylated on Thr68, and the data indicated that in this case 25% fewer AT cells showed basal damage after expressing GSE24.2 and high levels of damage to DNA were reduced by 13% (Figure 2). Therefore expression of GSE24.2 is able to correct damage to DNA in 28% of cells, confirming the data obtained with phosphorylated histone H2AX.

### EXAMPLE 2. Ability of GSE24.2 to reduce levels of free radicals in cells of patients with ataxia telangiectasia.

One of the characteristics of AT cells is an increase in free radical content. This is due to the failure of function of protein ATM, which is able to maintain the redox balance in normal cells when the cells are exposed to environmental stress such as *in vitro* culture. In order to verify if expression of GSE24.2 has some impact on the regulation of free radical levels, we used the compound dihydroethidium (Hydroethidine), which enables quantifying the levels of free radicals in living cells. For this experiment, we used control lymphoblasts AT736C and a line of AT lymphoblasts AT3189-P. The latter were infected with a lentiviral vector expressing GSE24.2, pCMV-GFP-GSE24.2, or an empty vector expressing only GFP (GFP). Three weeks after infection, the cells were loaded with the DHE reagent for 30 minutes and the proportion of cells with different free radical content was determined by flow cytometry. The results showed (Figure 3) that the level of free radicals in AT736C cells was less than in AT3189-P cells expressing the empty vector (GFP). By contrast, ROS levels reduced in AT3189-P GSE24.2 cells to values comparable to those found in the AT736C control cells.

### EXAMPLE 3. Ability of GSE24.2 to reduce levels of phosphorylated p38 kinase in cells of patients with ataxia telangiectasia.

One of the characteristics of AT patient cells is a higher activation of p38 MAP kinase due to the failure of function of the ATM protein. This increase in activation of p38 triggers the process of cell death in AT cells and therefore a reduction in the number of neuronal cells. Therefore we checked if the expression of GSE24.2 was able to reduce the activation of p38 in cell lines from AT patients. AT719 cells were infected with a lentiviral vector expressing GSE24.2, pCMV-GFP-GSE24.2, or an empty vector expressing only GFP (GFP) and protein extracts were obtained from both cells lines three weeks after infection. The normal lymphoblasts cell line AT736 was used as negative control. The extracts were resolved on polyacrylamide gels and the proteins transferred to nitrocellulose filters for subsequent hybridisation with antibodies. An antibody that detects phosphorylated p38 protein was used and later a rehybridisation was performed with an antibody against non-phosphorylated p38 protein as a loading control. The signal obtained in both hybridisations was quantified with the NIH-Image program and relative values between the phosphorylated and non-phosphorylated proteins obtained for the control cell line were considered as 1. Figure 4 shows that the valued increased much more in the case of AT719 cells infected with the GFP virus, but reduced significantly to levels similar to control cells when infected with the GSE24.2 virus. These data indicate that one of the consequences of the failure of activation of ATM and associated oxidative stress, the activation of p38, is reversed after the expression of GSE24.2.

### EXAMPLE 4. Ability of GSE24.2 to increase viability after treatment with a radiomimetic drug in cells from patients with ataxia telangiectasia.

Mutations in gene ATM sensitise cells of AT patients to treatment with ionizing radiation or radiomimetic drugs such as bleomycin. In order to verify if the protective effect induced by GSE24.2 in cells of AT patients also extends to radiomimetic agents, AT719 patient cells expressing pCMV-GFP-GSE24.2 or an empty vector expressing only GFP (GFP) were treated with increasing concentrations of bleomycin for 72 hours. After this time, cell viability was evaluated using MTS reagent. AT736C cells were used as a control. The results showed that expression of GSE24.2 was able to increase viability of AT719 cells to levels similar to control cells, whereas AT719 or AT719GFP cells were more sensitive than controls (Figure 5). These results show that the expression of GSE24.2 is able to protect from cell death induced by radiomimetic agents.

### EXAMPLE 5. Test of activity of the described peptides. Repair of damage to DNA.

Bacterial lysates were obtained from RosettaGami2 bacteria expressing the bacterial vector pGATEVGSE24-2. To do this, the bacteria were sonicated in saline medium with protease inhibitors and the fusion protein expressed by the bacterial plasmid was purified on glutathione sepharose columns. The lysates were subjected to HPLC to separate the peptides from the other cellular proteins using columns for separation by size and charge. The resulting fractions were sequenced using a sequencer that uses a method derived from the Edman method and in commercial use. The various peptides found in bacterial lysates of bacteria expressing GSE24.2 were obtained by chemical synthesis and tested for the activity of repairing damage to DNA using F9A353V cells carrying the most frequent mutation (A353V) in patients with congenital dyskeratosis. To do this, various peptides were lipofected using Proteojuice reagent in F9A353V cells and F9 control cells (cells without damage) were used for control of damage. An unrelated peptide was used as a negative control and the full GSE24.2 as a positive control. After quantifying the Western blots (Figure 6), it was found that GSE4 is the smallest sized peptide able to repair the damage to DNA measured by the amount of phosphorylation of histone H2AX Ser319 that appeared in the A353V cells (Figure 7).

In order to optimise the operating conditions of peptide GSE4, different amounts of peptide GSE4 were transfected into F9A353V cells and the amount of damage to DNA was determined using a specific antibody as was performed in Figure 6 (Figure 8). After quantification of the Western blot (Figure 9), maximum inhibition of damage to DNA was obtained by transfecting 8 µg of peptide GSE4.

### EXAMPLE 6. Measurement of the increase in telomerase activity after treatment with peptide GSE4.

The other way of quantifying the activity of peptide GSE4 was to evaluate its ability to increase telomerase activity in VA13 cells. To do this, these cells were transfected with 8 µg of the two peptides GSE4 and GSE5. After 48 hours, the cells were processed to test the telomerase activity by the TRAP method.

Figure 10 (A and B) shows that although peptide GSE5 has a repair activity for damage to DNA similar to that of GSE4, the latter has a much higher activity in reactivating telomerase activity. Therefore the activity of peptide GSE4 is more effective for reactivating telomerase activity.

### EXAMPLE 7. Measurement of the reduction in oxidative stress, inflammation and senescence after treatment with peptide GSE4.

As the peptide denominated GSE4 was smaller than the peptides with ability to repair telomerase activity, we studied if it had the same activity as peptide GSE24.2 for attenuating oxidative stress, expression of proinflammatory cytokines and repairing the senescence in ataxia telangiectasia cells. Figure 11 shows that peptide GSE4 was able to reduce the levels of free radicals by 50%, similarly to peptide GSE24.2, in AT719-P cells. In addition, it was able to reduce the levels of expression of IL6 and IL8 by 80% and 90% respectively in these cells (Figure 12), these values being in the same range as for peptide GSE24.2. Finally, given that the increase in expression of the interleukins IL6 and IL8 are associated with a secretory phenotype linked to senescence in ataxia telangiectasia, we evaluated the ability of both peptides GSE24.2 and GSE4 to reduce senescence by evaluating senescence-associated β-galactosidase activity (SA-beta-Gal) (Figure 13). The data showed that the expression of both peptides reduced the percentage of positive cells for beta-galactosidase activity progressively over time. The results of these experiments show that the sequence of peptide GSE4 conserves the region necessary, in addition to reducing the damage to DNA and increasing telomerase activity, for reducing oxidative stress, proinflammatory interleukin levels and senescence.

### EXAMPLE 8. Measurement of damage to DNA with peptide GSE4 and a peptide with a similar sequence localised in the TRUBII domain of dyskerin denominated SGSE4.

As the peptide denominated GSE4 was the smallest sized of the peptides with ability to inhibit damage to DNA, we studied if another peptide with a similar sequence localised in the sequence of peptide GSE24.2 had the same activity or if that activity was specific to GSE4. Peptide SGSE4 (SEQ ID No. 15) is similar to GSE4 in sequence (amino acids LDPK/LDKP that constitute consensus sequences for pseudouridine synthases) and size and is found in the TRUBII domain of dyskerin. Cell lines AT2078 of patients with ataxia telangiectasia were used for this test. Cell line C-1787 was used as the control cell line. The cells were infected with the bicistronic lentiviral vector pCMV-GFP-GSE4, SGSE4 or GFP. After 48 hours of infection the cells were fixed with formaldehyde and then permeabilized by treating them with Triton X-100. The damage to DNA was estimated by studying the signal after hybridisation of the cells with an antibody against histone H2AX Ser139 and subsequent hybridisation with an antibody bound to fluorescein. The number of foci with stain for histone H2AX per nucleus was determined by confocal microscopy in a total of 200 cells and the data represented as a percentage of cells with a specific number of foci.

Figure 14 shows that the expression of GSE4 corrected the overall damage to DNA in 38% of the AT cells, so an overall correction of damage to DNA was of 60%. Peptide SGSE4 was not able to correct the damage to DNA in the same conditions. Therefore, only the sequence of GSE4 and no other similar is able to repair the damage to DNA in AT cells.

### EXAMPLE 9. Test of the ability of peptide GSE24.2 with and without nuclear localisation signal to enter cells.

An important aspect to be considered for peptide GSE24.2 is its subcellular localisation after it is expressed. Therefore various constructions were obtained, placing two nuclear localisation signals NLS1 and NLS2 in the 5' and 3' region of GSE24.2. NLS1 is a signal present in the sequence of gene DKC1 that carries the protein to the nucleus and sequence NLS2 mediates transport to the nucleolus. Figure 15 shows the sequence of both nuclear localisation sequences.

The expression plasmid in eukaryotic cells, pCDNA3myc, was used, which contains a tag of the gene c-myc in position 5' merged with the sequence GSE24.2. Various constructions were obtained that are shown in Figure 16, placing sequences NLS1 and NLS2 in both 5' and 3' positions in the sequence of GSE24.2. The constructions denominated NLS1-3, NLS1-5, NLS2-3 and NLS2-5 of GSE24.2 were transfected into HeLa cells. These constructions carried the tag sequence of c-myc recognised with antibody 9E10 to localise the expression of the constructions.

Figure 17 shows that the constructions with localisation signals NLS1 or NLS2 in the 3' position of GSE24.2 were those that preferentially facilitated expression of GSE24.2 in the nucleus. Figures 18 and 19 show a detail of the subcellular localisation of constructions NLS1-3 and NLS2-3.

### EXAMPLE 10. Activity of constructions GSE24.2NLS1-3 and GSE24.2NLS2-3 to activate the expression of c-myc.

With the aim of evaluating biological activity of the constructions containing the signals NLS1-3 and NLS2-3, they were transfected into 293T cells together with the reporter gene of the promoter of c-myc and the luciferase activity activated by both constructions GSE24.2NLS1-3 and GSE24.2NLS2-3 was evaluated in relation to GSE24.2. Figure 20 shows that vectors GSE24.2NLS1-3 and GSE24.2NLS2-3 activated the expression of the promoter of c-myc more effectively than GSE24.2. These data are consistent with a higher ability of both constructions to be expressed in the cell nucleus. Out of the two constructions, the most effective was the construction with the NLS1 signal in the 3' position.

### EXAMPLE 11. Ability of GSE4 to enter cells.

Given the ability of sequence NLS1 to contribute to the activity of GSE24.2, a synthetic peptide into which this sequence was placed in the 3' region of peptide GSE4 was constructed as this was the peptide for which the greatest telomerase activation was obtained. The peptides obtained by chemical synthesis were labelled with fluorescein to facilitate their visualisation in a fluorescence microscope. Two experiments were performed in parallel, in one the peptides were transfected using Proteojuice reagent and in the other they were added directly to the culture medium. The results showed that both GSE4 and GSE4NLS1 (SEQ ID No.7) entered the cells by using Proteojuice reagent (Figure 21) or by simply adding them to the culture medium (Figure 22). An important difference observed was that when the peptides were added directly to the culture medium, the peptide entered into a higher number of cells than when Proteojuice was used. The reagent provided greater effectiveness for internalisation but into a small number of cells.

## Claims

1. Polypeptide **characterised in that** it comprises a fragment of the sequence of peptide GSE24.2 (SEQ ID No. 1) with SEQ ID No. 2 able to reduce production of free radicals and/or damage to the structure of cellular DNA and said polypeptide is selected from the list consisting of: SEQ ID No. 2, SEQ ID No. 5 and SEQ ID No. 6.

2. Polypeptide according to claim 1 **characterised in that** further comprises at least one nuclear localisation sequence bound to at least one of the carboxyl or amino terminal ends thereof.

3. Polypeptide according to claim 2 **characterised in that** the nuclear localisation sequence is the SEQ ID NO: 16 and/or the SEQ ID NO: 17.

4. Polypeptide according to any of the claims 2 to 3 **characterised in that** it consists of one sequence selected from the group composed of: SEQ ID No. 7, SEQ ID No. 12, SEQ ID No. 13 and SEQ ID No. 14.

5. Polynucleotide **characterised in that** it codes for a polypeptide defined in any of the claims 1 to 4.

6. Expression vector **characterised in that** it comprises a polynucleotide sequence that codes for a polypeptide defined in any of the claims 1 to 4.

7. Pharmaceutical composition **characterised in that** it comprises at least a polypeptide defined in any of claims 1 to 4 and/or a polynucleotide of claim 5 and/or expression vector of claim 6.

8. Pharmaceutical composition according to claim 7 **characterised in that** it further comprises at least one pharmaceutically acceptable adjuvant and/or vehicle.

9. Polypeptide according to any one of the claims 1 to 4 for use in the treatment and/or prevention of a disease and/or disorder caused by an increase in oxidative stress and/or damage to cellular DNA.

10. Polypeptide for use according to claim 9 **characterised in that** the polypeptide is selected from at least one of the group composed of: SEQ ID No. 2, SEQ ID No. 5, SEQ ID No. 6, SEQ ID No. 7, SEQ ID No. 12, SEQ ID No. 13, and SEQ ID No. 14.

11. Polynucleotide according to claim 5 for use in the treatment and/or prevention of a disease and/or a disorder caused by an increase in oxidative stress and/or damage to cellular DNA.

12. An expression vector according to claim 6 for use in the treatment and/or prevention of a disease and/or a disorder caused by an increase in oxidative stress and/or damage to cellular DNA.

13. Polypeptide for use according to claims 9 and 10 , polynucleotide for use according to claim 5 or expression vector for use according to claim 6, **characterised in that** the disease or disorder is selected from at least one of the group composed of: congenita dyskeratosis, Cri du Chat syndrome, ataxia telangiectasia, Nijmegen breakage syndrome, Bloom syndrome, Werner syndrome, Fanconi anaemia, ulcerative colitis, vascular ageing, arteriosclerosis, atherosclerosis, cancer, Duchenne muscular dystrophy, progeria, light hypersensitivity, genetic instability caused by mutation or external agent, Hutchison-Gilford syndrome, xeroderma pigmentosum, Rothmund-Thomson syndrome, pulmonary fibrosis, rheumatoid arthritis, a disease with chronic inflammation and a neurodegenerative disease selected from at least one of the group composed of: Huntington's disease, Alzheimer's disease, Parkinson's disease, cerebellar ataxia and spinal medulla degeneration.

14. Use *in vitro* of a polypeptide according to anyone of claims 1 to 4, or of the polynucleotide according to claim 5, or the vector according to claim 6 in tissue engineering and cell culture for improving the cell viability.

## Patentansprüche

1. Polypeptid, **dadurch gekennzeichnet, dass** es ein Fragment der Sequenz von Peptid GSE24.2 (SEQ ID Nr. 1) mit SEQ ID Nr. 2 umfasst, das die Herstellung von freien Radikalen und/oder Schädigung der Struktur von Zell-DNA reduzieren kann und das Polypeptid ausgewählt ist aus der Liste, bestehend aus: SEQ ID Nr. 2, SEQ ID Nr. 5 und SEQ ID Nr. 6.

2. Polypeptid nach Anspruch 1, **dadurch gekennzeichnet, dass** es ferner mindestens eine Kernlokalisierungssequenz umfasst, die an mindestens eines der carboxyl- oder aminoterminierten Enden davon gebunden ist.

3. Polypeptid nach Anspruch 2, **dadurch gekennzeichnet, dass** die Kernlokalisierungssequenz die SEQ ID Nr. 16 und/oder die SEQ ID Nr. 17 ist.

4. Polypeptid nach einem der Ansprüche 2 bis 3, **dadurch gekennzeichnet, dass** es aus einer Sequenz besteht, die ausgewählt ist aus der Gruppe, zusammengesetzt aus: SEQ ID Nr. 7, SEQ ID Nr. 12, SEQ ID Nr. 13 und SEQ ID Nr. 14.

5. Polynukleotid, **dadurch gekennzeichnet, dass** es ein Polypeptid codiert, das in einem der Ansprüche 1 bis 4 definiert ist.

6. Expressionsvektor, **dadurch gekennzeichnet, dass** er eine Polynukleotidsequenz umfasst, die ein Polypeptid codiert, das in einem der Ansprüche 1 bis 4 definiert ist.

7. Pharmazeutische Zusammensetzung, **dadurch gekennzeichnet, dass** diese mindestens ein Polypeptid umfasst, das in einem der Ansprüche 1 bis 4 definiert ist, und/oder ein Polynukleotid nach Anspruch 5 und/oder einen Expressionsvektor nach Anspruch 6.

8. Pharmazeutische Zusammensetzung nach Anspruch 7, **dadurch gekennzeichnet, dass** diese ferner mindestens einen pharmazeutisch verträglichen Hilfsstoff und/oder Trägerstoff umfasst.

9. Polypeptid nach einem der Ansprüche 1 bis 4 zur Verwendung bei der Behandlung und/oder Vorbeugung einer Krankheit und/oder Störung, die durch eine Erhöhung des oxidativen Stresses und/oder Beschädigung der Zell-DNA verursacht wird.

10. Polypeptid zur Verwendung nach Anspruch 9, **dadurch gekennzeichnet, dass** das Polypeptid ausgewählt ist aus mindestens einem von der Gruppe, die zusammengesetzt ist aus: SEQ ID Nr. 2, SEQ ID Nr. 5, SEQ ID Nr. 6, SEQ ID Nr. 7, SEQ ID Nr. 12, SEQ ID Nr. 13 und SEQ ID Nr. 14.

11. Polynukleotid nach Anspruch 5 zur Verwendung bei der Behandlung und/oder Vorbeugung einer Krankheit und/oder einer Störung, die durch eine Erhöhung des oxidativen Stresses und/oder Beschädigung der Zell-DNA verursacht wird.

12. Expressionsvektor nach Anspruch 6 zur Verwendung bei der Behandlung und/oder Vorbeugung einer Krankheit und/oder einer Störung, die durch eine Erhöhung des oxidativen Stresses und/oder Beschädigung der Zell-DNA verursacht wird.

13. Polypeptid zur Verwendung nach einem der Ansprüche 9 und 10, Polynukleotid zur Verwendung nach Anspruch 5 oder Expressionsvektor zur Verwendung nach Anspruch 6, **dadurch gekennzeichnet, dass** die Krankheit oder Störung ausgewählt ist aus mindestens einem von der Gruppe, die zusammengesetzt ist aus: Dyskeratosis Congenita, Katzenschreisyndrom, Ataxie-Telangiektasie, Nijmegen-Breakage-Syndrom, Bloom-Syndrom, Werner-Syndrom, Fanconi-Anämie, Colitis ulcerosa, Gefäßalterung, Arteriosklerose, Atherosklerose, Krebs, Duchenne-Muskeldystrophie, Progerie, Licht-Überempfindlichkeit, genetische Instabilität aufgrund durch Mutation oder externe Wirkstoffe, Hutchison-Gilford-Syndrom, Pigmentxerodermie, Rothmund-Thomson-Syndrom, Lungenfibrose, rheumatoide Arthritis, eine Erkrankung mit chronischer Entzündung und einer neurodegenerativen Krankheit, ausgewählt aus mindestens einem von der Gruppe, zusammengesetzt aus: Huntingtons Krankheit, Alzheimerkrankheit, Parkinsonkrankheit, zerebellare Ataxie und Gehirnstrangataxie.

14. *In-Vitro*-Verwendung eines Polypeptids nach einem der Ansprüche 1 bis 4 oder des Polynukleotids nach Anspruch 5 oder des Vektors nach Anspruch 6 in der Gewebetechnik und Zellkultur zum Verbessern der Zelllebensfähigkeit.

## Revendications

1. Polypeptide **caractérisé en ce qu'**il comprend un fragment de la séquence de peptide GSE24.2 (SEQ ID No. 1) avec SEQ ID No. 2 capable de réduire la production de radicaux libres et/ou les dommages à la structure d'ADN cellulaire et ledit polypeptide est choisi dans la liste consistant en : SEQ ID No. 2, SEQ ID No. 5 et SEQ ID No. 6.

2. Polypeptide selon la revendication 1 **caractérisé en ce qu'**il comprend en outre au moins une séquence de localisation nucléaire liée à au moins l'une de ses extrémités carboxyle ou amino-terminale.

3. Polypeptide selon la revendication 2 **caractérisé en ce que** la séquence de localisation nucléaire est la SEQ ID NO: 16 et/ou la SEQ ID NO: 17.

4. Polypeptide selon l'une quelconque des revendications 2 à 3 **caractérisé en ce qu'**il consiste en une séquence choisie dans le groupe composé de : SEQ ID No. 7, SEQ ID No. 12, SEQ ID No. 13 et SEQ ID No. 14.

5. Polynucléotide **caractérisé en ce qu'**il code pour un polypeptide défini dans l'une quelconque des revendications 1 à 4.

6. Vecteur d'expression **caractérisé en ce qu'**il comprend une séquence polynucléotidique qui code pour un polypeptide défini dans l'une quelconque des revendications 1 à 4.

7. Composition pharmaceutique **caractérisée en ce qu'**elle comprend au moins un polypeptide défini dans l'une quelconque des revendications 1 à 4 et/ou un polynucléotide de la revendication 5 et/ou un vecteur d'expression de la revendication 6.

8. Composition pharmaceutique selon la revendication 7 **caractérisée en ce qu'**elle comprend en outre au moins un adjuvant et/ou véhicule pharmaceutiquement acceptable.

9. Polypeptide selon l'une quelconque des revendications 1 à 4 pour son utilisation dans le traitement et/ou la prévention d'une maladie et/ou d'un trouble provoqués par une augmentation du stress oxydatif et/ou des dommages à l'ADN cellulaire.

10. Polypeptide pour son utilisation selon la revendication 9 **caractérisé en ce que** le polypeptide est choisi parmi au moins l'un du groupe composé de : SEQ ID No. 2, SEQ ID No. 5, SEQ ID No. 6, SEQ ID No. 7, SEQ ID No. 12, SEQ ID No. 13, et SEQ ID No. 14.

11. Polynucléotide selon la revendication 5 pour son utilisation dans le traitement et/ou la prévention d'une maladie et/ou d'un trouble provoqués par une augmentation du stress oxydatif et/ou des dommages à l'ADN cellulaire.

12. Vecteur d'expression selon la revendication 6 pour son utilisation dans le traitement et/ou la prévention d'une maladie et/ou d'un trouble provoqués par une augmentation du stress oxydatif et/ou des dommages à l'ADN cellulaire.

13. Polypeptide pour son utilisation selon les revendications 9 et 10, polynucléotide pour son utilisation selon la revendication 5 ou vecteur d'expression pour son utilisation selon la revendication 6, **caractérisé en ce que** la maladie ou le trouble est choisi parmi au moins l'un du groupe composé de : dyskératose congénitale, Syndrome de Cri du Chat, ataxie télangiectasie, Syndrome de Nimègue, Syndrome de Bloom, Syndrome de Werner, Anémie de Fanconi, colite ulcéreuse, vieillissement vasculaire, artériosclérose, athérosclérose, cancer, Dystrophie musculaire de Duchenne, progéria, hypersensibilité à la lumière, instabilité génétique causée par une mutation ou un agent externe, Syndrome de Hutchison-Gilford, xeroderma pigmentosum, Syndrome de Rothmund-Thomson, fibrose pulmonaire, arthrite rhumatoïde, une maladie avec inflammation chronique et une maladie neurodégénérative choisies parmi au moins l'un du groupe composé de : maladie de Hurtington, maladie d'Alzheimer, maladie de Parkinson, ataxie cérébelleuse et dégénérescence de la moelle épinière.

14. Utilisation *in vitro* d'un polypeptide selon l'une quelconque des revendications 1 à 4, ou du polynucléotide selon la revendication 5, ou du vecteur selon la revendication 6 en ingénierie tissulaire et culture cellulaire pour améliorer la viabilité cellulaire.


### LIST OF SEQUENCES

<110> Consejo Superior de Investigaciones Cientificas Universidad Autónoma de Madrid Advanced Medical Projects Centro de Investigación Biomedica en Red (CIBERER) Network
<120> PEPTIDES DERIVED FROM GSE24.2 FOR TREATING DISEASES CAUSED BY
   OXIDATIVE STRESS AND DAMAGE TO DNA
<130> 120140261
<150> ESP201331573
   <151> 2013-10-25
<160> 15
<170> BiSSAP 1.2
<210> 1
   <211> 55
   <212> PRT
   <213> Homo sapiens
<220>
   <223> GSE24.2
<400> 1
   Gly Phe Ile Asn Leu Asp Lys Pro Ser Asn Pro Ser Ser His Glu Val
   1               5                   10                  15
   Val Ala Trp Ile Arg Arg Ile Leu Arg Val Glu Lys Thr Gly His Ser
               20                  25                  30
   Gly Thr Leu Asp Pro Lys Val Thr Gly Cys Leu Ile Val Cys Ile Glu
           35                  40                  45
   Arg Ala Thr Arg Leu Val Lys
       50                  55
<210> 2
   <211> 11
   <212> PRT
   <213> Homo sapiens
<220>
   <223> GSE4
<400> 2
   Gly Phe Ile Asn Leu Asp Lys Pro Ser Asn Pro
   1               5                   10
<210> 3
   <211> 17
   <212> PRT
   <213> Homo sapiens
<220>
   <223> GSE1
<400> 3
   Gly Phe Ile Asn Leu Asp Lys Pro Ser Asn Pro Ser Ser His Glu Val
   1               5                   10                  15
   Val
<210> 4
   <211> 18
   <212> PRT
   <213> Homo sapiens
<220>
   <223> GSE2
<400> 4
   Gly Phe Ile Asn Leu Asp Lys Pro Ser Asn Pro Ser Ser His Glu Val
   1               5                   10                  15
   Val Ala
<210> 5
   <211> 19
   <212> PRT
   <213> Homo sapiens
<220>
   <223> GSE3
<400> 5
   Gly Phe Ile Asn Leu Asp Lys Pro Ser Asn Pro Ser Ser His Glu Val
   1               5                   10                  15
   Val Ala Trp
<210> 6
   <211> 25
   <212> PRT
   <213> Homo sapiens
<220>
   <223> GSE5
<400> 6
   Gly Phe Ile Asn Leu Asp Lys Pro Ser Asn Pro Ser Ser His Glu Val
   1               5                   10                  15
   Val Ala Trp Ile Arg Arg Ile Leu Arg
               20                  25
<210> 7
   <211> 15
   <212> PRT
   <213> Homo sapiens
<220>
   <223> GSE4NLS1-3
<400> 7
   Gly Phe Ile Asn Leu Asp Lys Pro Ser Asn Pro Lys Arg Lys Arg
   1               5                   10                  15
<210> 8
   <211> 59
   <212> PRT
   <213> Homo sapiens
<220>
   <223> GSE24.2NLS1-3
<400> 8
   Gly Phe Ile Asn Leu Asp Lys Pro Ser Asn Pro Ser Ser His Glu Val
   1               5                   10                  15
   Val Ala Trp Ile Arg Arg Ile Leu Arg Val Glu Lys Thr Gly His Ser
               20                  25                  30
   Gly Thr Leu Asp Pro Lys Val Thr Gly Cys Leu Ile Val Cys Ile Glu
           35                  40                  45
   Arg Ala Thr Arg Leu Val Lys Lys Arg Lys Arg
       50                  55
<210> 9
   <211> 63
   <212> PRT
   <213> Homo sapiens
<220>
   <223> GSE24.2NLS2-3
<400> 9
   Gly Phe Ile Asn Leu Asp Lys Pro Ser Asn Pro Ser Ser His Glu Val
   1               5                   10                  15
   Val Ala Trp Ile Arg Arg Ile Leu Arg Val Glu Lys Thr Gly His Ser
               20                  25                  30
   Gly Thr Leu Asp Pro Lys Val Thr Gly Cys Leu Ile Val Cys Ile Glu
           35                  40                  45
   Arg Ala Thr Arg Leu Val Lys Lys Lys Glu Lys Lys Lys Ser Lys
       50                  55                  60
<210> 10
   <211> 59
   <212> PRT
   <213> Homo sapiens
<220>
   <223> GSE24.2NLS1-5
<400> 10
   Lys Arg Lys Arg Gly Phe Ile Asn Leu Asp Lys Pro Ser Asn Pro Ser
   1               5                   10                  15
   Ser His Glu Val Val Ala Trp Ile Arg Arg Ile Leu Arg Val Glu Lys
               20                  25                  30
   Thr Gly His Ser Gly Thr Leu Asp Pro Lys Val Thr Gly Cys Leu Ile
           35                  40                  45
   Val Cys Ile Glu Arg Ala Thr Arg Leu Val Lys
       50                  55
<210> 11
   <211> 63
   <212> PRT
   <213> Homo sapiens
<220>
   <223> GSE24.2NLS2-5
<400> 11
   Lys Lys Glu Lys Lys Lys Ser Lys Gly Phe Ile Asn Leu Asp Lys Pro
   1               5                   10                  15
   Ser Asn Pro Ser Ser His Glu Val Val Ala Trp Ile Arg Arg Ile Leu
               20                  25                  30
   Arg Val Glu Lys Thr Gly His Ser Gly Thr Leu Asp Pro Lys Val Thr
           35                  40                  45
   Gly Cys Leu Ile Val Cys Ile Glu Arg Ala Thr Arg Leu Val Lys
               50                  55                  60
<210> 12
   <211> 15
   <212> PRT
   <213> Homo sapiens
<220>
   <223> GSE4NLS1-5
<400> 12
   Lys Arg Lys Arg Gly Phe Ile Asn Leu Asp Lys Pro Ser Asn Pro
   1               5                   10                  15
<210> 13
   <211> 19
   <212> PRT
   <213> Homo sapiens
<220>
   <223> GSE4NLS2-3
<400> 13
   Gly Phe Ile Asn Leu Asp Lys Pro Ser Asn Pro Lys Lys Glu Lys Lys
   1               5                   10                  15
   Lys Ser Lys
<210> 14
   <211> 19
   <212> PRT
   <213> Homo sapiens
<220>
   <223> GSE4NLS2-5
<400> 14
   Lys Lys Glu Lys Lys Lys Ser Lys Gly Phe Ile Asn Leu Asp Lys Pro
   1               5                   10                  15
   Ser Asn Pro
<210> 15
   <211> 11
   <212> PRT
   <213> Homo sapiens
<400> 15
   His Ser Gly Thr Leu Asp Pro Lys Val Thr Gly
   1               5                   10